(19) Europäisches Patentamt
European Patent Office
Office européen des brevets

(11) Veröffentlichungsnummer: **0 102 318**
**B1**

(12) # EUROPÄISCHE PATENTSCHRIFT

(45) Veröffentlichungstag der Patentschrift: **14.01.87**

(21) Anmeldenummer: **83810302.6**

(22) Anmeldetag: **04.07.83**

(51) Int. Cl.⁴: **C 07 C 99/00**, C 07 C 101/28, C 07 C 121/78, C 07 D 521/00, C 09 B 57/00

(54) **Herstellung von beta-Amino-alpha,beta-ungesättigten Carbonsäureestern.**

(30) Priorität: **06.07.82 CH 4170/82**

(43) Veröffentlichungstag der Anmeldung: **07.03.84 Patentblatt 84/10**

(45) Bekanntmachung des Hinweises auf die Patenterteilung: **14.01.87 Patentblatt 87/3**

(84) Benannte Vertragsstaaten: **CH DE FR GB IT LI**

(73) Patentinhaber: **CIBA- GEIGY AG, Klybeckstrasse 141, CH- 4002 Basel (CH)**

(72) Erfinder: **Cassar, Luigi, Dr., Via Orfeo 25, Bologna (IT)**
Erfinder: **Iqbal, Abul, Dr., Im Schaiengarten 1, CH- 4107 Ettingen (CH)**
Erfinder: **Rochat, Alain Claude, Dr., Schiffenenstrasse 38, CH- 1700 Fribourg (CH)**

(56) Entgegenhaltungen:
**EP-A-0 061 426**

**TETRAHEDRON LETTERS, Nr. 27, 1979, Seiten 2525-2528, Pergamon Press Ltd., GB, T. IIMORI et al.: "Carbon-carbon bond formation from ethyl cyanoformate and active methylene compounds"**
**BULLETIN DE LA SOCIETE CHIMIQUE DE FRANCE, 1972, Nr. 10, Seiten 3841-3846, Paris, FR., F. DARDOIZE et al.: "Réaction de Reformatsky sur les bases de Schiff. I - Préparation de beta-aminoesters"**
**TETRAHEDRON LETTERS, nr. 29, 1974, Seiten 2549-2552, Oxford, GB., D.G. FARNUM et al.: "Attempted Reformatskii reaction of benzonitrile, 1,4-diketo-3,6-diphenylpyrrolo(3,4-C)pyrrole. A lactam analogue of pentalene"**
**BULLETIN DE LA SOCIETE CHIMIQUE DE FRANCE, 1966, Seiten 1819-1822, Paris, FR., H.B. KAGAN et al.: "Réaction de Réformatsky sur les nitriles. I. - Préparation de beta-céto-esters non substitués, mono ou disubstitués en alpha"**
**BULLETIN DE LA SOCIETE CHIMIQUE DE FRANCE, 1968, Seiten 3403-3408, Paris, FR., L. ARSENIJEVIC et al.: "Réaction de Réformatsky sur les nitriles-esters aromatiques. I. - Synthese des hydroxy-1 isoquinoléines et des hydroxy-3 isoquinoléines substituées"**
**SYNTHESIS, Oktober 1977, Seite 698, Stuttgart, DE., E. SANTANIELLO et al.: "Use of the Zn-Cu Couple in the Reformatsky Reaction"**

EP 0 102 318 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Zur Herstellung von β-amino-α,β-ungesättigten Carbonsäureestern sind verschiedene Verfahren bekannt. So wird beispielsweise im Journal of Heterocyclic Chemistry 15, 1001 (1978) die Herstellung von β-Aminozimtsäureäthylester ausgehend von Cyanoessigsäureäthylester und einem Phenylmagnesiumhalogenid beschrieben. Auch in Bulletin de la Société Chimique de France, 1966, Seiten 1819-1822 werden β-amino-α,βungesättigte Carbonsäureester beschrieben. Dort sind sie aber bei der Herstellung von ß-Ketoestern ausgehend von einem Nitril, einem α-Bromessigester und Zink als Zwischenprodukte bzw. Nebenprodukte aufgeführt. Ferner wird in der EP-Patentanmeldung Nr. 0061426 ein Verfahren ausgehend von einem aromatischen Nitril, Bromessigester und Zink beschrieben. Dabei wird ein Pyrrolo-pyrrol erhalten. Die nach den bekannten Verfahren erzielten Ausbeuten an ß-amino-α,ß-ungesättigten Carbonsäureestern sind ungenügend. Wählt man hingegen als Ausgangsstoffe ein aromatisches Nitril und einen Halogenessigsäureester zusammen mit besonderen Reaktionsbedingungen, so erhält man auf sehr einfache, wirtschaftliche Weise, selektiv und in hoher Ausbeute ß-amino-α,ß-ungesättigte Carbonsäureester. Dies ist überraschend, da gemäss Tetrahedron Letters 23, 1597-1600 (1982) bisher vergeblich versucht wurde, einen β-amino-α,β-ungesättigten Carbonsäureester aus Bromessigsäureäthylester und Benzonitril herzustellen.

Die Erfindung betrifft somit ein Verfahren zur Herstellung von Verbindungen der Formel I

$$R_1 - \underset{NH_2}{\underset{|}{C}} = \underset{R_2}{\underset{|}{C}} - COOA \qquad (I),$$

worin A Alkyl oder Aryl bedeutet, $R_1$ einen isocyclischen oder hererocyclischen aromatischen Rest bedeutet. $R_2$ Wasserstoff, Alkyl, Aryl, Cyano oder die Gruppe -COOZ bedeutet, worin Z Alkyl oder Aryl ist, indem man 1 Mol eines aromatischen Nitrils der Formel

$$R_1 - CN \ (II)$$

mit 1 Mol eines Essigesters der Formel III

$$X - \underset{R_2}{\underset{|}{CH}} - COOA \qquad (III),$$

worin X Halogen bedeutet, in Gegenwart von Zink in einem inerten organischen Lösungsmittel umsetzt, dadurch gekennzeichnet, dass die Umsetzung bei einer Temperatur von weniger als 80°C durchgeführt wird, und dass das entstandene Reaktionsprodukt alkalisch hydrolysiert und die Verbindung der Formel 1 z.B. durch Extraktion isoliert wird.

Bedeutet $R_1$ einen isocyclischen aromatischen Rest, dann vorzugsweise einen mono- bis tetra-cyclischen, insbesondere mono- oder bicyclischen Rest, also Phenyl, Diphenylyl oder Naphthyl. Bedeutet $R_1$ einen heterocyclischen aromatischen Rest, dann vorzugsweise einen monobis tricyclischen. Dieser kann rein heterocyclisch sein oder einen heterocyclischen Ring und einen oder mehrere ankondensierte Benzolringe enthalten, wie z.B. Thienyl, Furyl, Pyrrolyl, Thiophenyl, Pyridyl, Chinolyl, Cumarinyl, Benzfuranyl, Benzimidazolyl oder Benzoxazolyl. Sowohl die isocyclischen wie die heterocyclischen aromatischen Reste können Substituenten aufweisen, wie:

1) Halogenatome, beispielsweise Chlor, Brom oder Fluor.

2) Verzweigte oder unverzweigte Alkylgruppen mit vorzugsweise 1 bis 18, insbesondere 1 bis 12, vor allem 1 bis 8 und besonders bevorzugt mit 1 bis 4 C-Atomen. Diese Alkylgruppen können Substituenten aufweisen, wie beispielsweise Fluor, Cyano, $-OCOR_3$, $-OR_4$, $-COOR_3$ oder $-CONR_4R_5$, worin $R_3$ Alkyl, Aryl, wie Phenyl oder Napthyl, unsubstituiertes oder durch Halogen, Alkyl oder -O-Alkyl substituiertes Benzyl oder einen heterocyclischen Rest bedeuten, $R_4$ und $R_5$ unsubstituiertes oder durch Cyano substituiertes Alkyl, $C_5$-$C_6$-Cycloalkyl, Aryl oder Heteroaryl bedeuten, oder worin $R_4$ und $R_5$ zusammen mit dem N-Atom einen 5-6-gliedrigen Heteroring bilden, wie beispielsweise einen Morpholin-, Piperidin- oder Phthalimidring. Weitere mögliche Substituenten an den Alkylgruppen sind dialkylierte Aminogruppen, Arylreste, wie Naphthyl, oder insbesondere unsubstituiertes oder durch Halogen, Alkyl oder -0-Alkyl substituiertes Phenyl, oder ferner heterocyclische aromatische Reste, wie z.B. die 2-Thienyl-, 2-Benzoxazolyl- 2-Benzthiazolyl-, 2-Benzimidazolyl-, 6-Benzimid-azolonyl-, 2-, 3- oder 4-Pyridyl-, 2-, 4- oder 6-Chinolylreste.

Enthalten die unter 2) genannten Substituenten ihrerseits wieder Alkyl, so kann dieses Alkyl verzweigt oder unverzweigt sein und vorzugsweise 1 bis 18, insbesondere 1 bis 12, vor allem 1 bis 8 und besonders bevorzugt 1 bis 4 C-Atome enthalten.

Beispiele von unsubstituierten oder substituierten Alkylgruppen sind Methyl, Aethyl, n-Propyl, Isopropyl, n-Butyl, sec.-Butyl, tert.-Butyl, tert.-Amyl, n-Pentyl, n-Hexyl, 1,1,3,3-Tetramethylbutyl, n-Heptyl, n-Octyl, Nonyl, Decyl, Undecyl, Dodecyl, Trifluormethyl, Trifluoräthyl, Cyanmethyl, Methoxycarbonylmethyl, Acetoxymethyl oder Benzyl.

3) Die Gruppe $-OR_6$, worin $R_6$ Alkyl, Aryl, beispielsweise Naphthyl, oder insbesondere unsubstituiertes oder durch Halogen, Alkyl, oder -O-Alkyl substituiertes Phenyl, $C_5$-$C_6$-Cycloalkyl, Aralkyl oder einen heterocyclischen Rest bedeutet. In den Definitionen von $R_6$ vorkommendes Alkyl kann z.B. eine der unter 2) als bevorzugt angegebenen Anzahl C-Atome haben. Als Beispiele von $R_6$ seien genannt: Methyl, Aethyl, n-Propyl, Isopropyl, Trifluoräthyl, Phenyl, o-, m- oder p-Chlorphenyl, o-, m- oder p-Methylphenyl, α- oder β-Naphthyl, Cyclohexyl, Benzyl, Thienyl oder Pyranylmethyl.

4) Die Gruppe $-SR_6$, worin $R_6$ die unter 3) angegebene Bedeutung hat. Als Beispiele für $R_6$ seien genannt: Methyl, Aethyl, n-Propyl, Isopropyl, Phenyl, o-, m- oder-p-Chlorphenyl, o-, m- oder p-Methylphenyl, α- oder β-Naphthyl, Cyclohexyl, Benzyl, Thienyl oder Pyranylmethyl.

5) Die Cyangruppe.

6) Die Gruppe der Formel $-NR_4R_5$, worin $R_4$ und $R_5$ die unter 2) angegebene Bedeutung haben. Als Beispielen seien genannt: Dimethylamino, Diäthylamino, N,N-Bis-(β-cyanäthyl)-amino, N-Methylphenylamino, Dibenzylamino, Piperidyl oder Morpholyl.

7) Die Gruppe der Formel $-COOR_3$, worin $R_3$ die unter 2) angegebene Bedeutung hat. Als Beispiele für $R_3$ seien genannt: Methyl, Aethyl, Isopropyl, n-Butyl, tert.-Butyl, Phenyl, Benzyl oder Furfuryl.

8) Die Gruppe der Formel $-NR_7COR_3$, worin $R_3$ die unter 2) angegebene Bedeutung hat, $R_7$ Alkyl, Aryl, beispielsweise Naphthyl oder insbesondere unsubstituiertes oder durch Halogen, Alkyl oder -O-Alkyl substituiertes Phenyl, $C_5$-$C_6$-Cycloalkyl, Aralkyl oder den Rest $-COR_3$ bedeutet, wobei zwei Reste $-COR_3$ zusammen mit dem N-Atom einen heterocyclischen Ring bilden können. In den Definitionen von $R_7$ vorkommendes Alkyl kann z.B. eine der unter 2) als bevorzugt angegebenen Anzahl C-Atome haben. Als Beispiele seien genannt:. N-Methylacetylamino, N-Methyl-benzoylamino, N-Succinimido oder N-Phthalimido.

9) Die Gruppe der Pormel $-NR_6COOR_3$, worin $R_3$ und $R_6$ die unter 2) bzw. 3) angegebene Bedeutung haben. Als Beispiele seien die Gruppen $-N(CH_3)CCOCH_3$, $-N(Phenyl)COOC_2H_5$ oder $-N(CH_3)COC_6H_5$ genannt.

10) Die Gruppe der Formel $-NR_6CONR_4R_5$, worin $R_6$, $R_5$ und $R_4$ die unter 3) und 2) angegebene Bedeutung haben. Als Beispiele seien genannt: N,N,N'-Trimethyl-ureido oder N,N-Dimethyl-N'-phenylureido.

11) Die Gruppen der Formel $-SO_2R_3$ oder $-SOR_3$, worin $R_3$ die unter 2) angegebene Bedeutung hat. Als Beispiele seien genannt: Methylsulfonyl, Aethylsulfonyl, Phenylsulfonyl, 2-Naphthylsulfonyl, Phenylsulfoxidyl.

12) Die Gruppe der Formel $-SO_2OR_3$, worin $R_3$ die unter 2) angegebene Bedeutung hat. Als Beispiele für $R_3$ seien genannt: Methyl, Aethyl, Phenyl o- m- oder p-Chlorphenyl, o-, m- oder p-Methylphenyl, αoder β-Naphthyl.

13) Die Gruppe der Formel $-CONR_4R_5$, worin $R_4$ und $R_5$ die unter 2) angegebene Bedeutung haben. Als Beispiele seien genannt: N,N-Dimethylcarbamoyl, N-Methyl-N-Phenylcarbamoyl oder N-Piperidylcarbamoyl.

14) Die Gruppe der Formel $-SO_2NR_4R_5$, worin $R_4$ und $R_5$ die unter 2) angegebene Bedeutung haben. Als Beispiele seien genannt: N-Methyl-Nphenylsulfamoyl oder N-Morpholylsulfamoyl.

15) Die Gruppe der Formel $-N{=}N-R_8$, worin $R_8$ den Rest einer Kupplungskomponente oder einen gegebenenfalls durch Halogen, Alkyl oder -0-Alkyl substituierten Phenylrest bedeutet. In den Definitionen von $R_8$ vorkommendes Alkyl kann z.B. eine der unter 2) als bevorzugt angegebenen Anzahl C-Atome haben. Als Beispiele für $R_8$ seien genannt: Die Pyrazolyl-, Pyridonyl- oder p-N,N-Dimethylaminophenylreste.

16) Die Gruppe der Pormel $-OCOR_3$, worin $R_3$ die unter 2) angegebene Bedeutung hat. Als Beispiele für $R_3$ seien genannt: Methyl, Aethyl, Phenyl, o-, m- oder p-Chlorphenyl.

Bedeuten A, $R_2$ und Z Alkyl, so kann dieses verzweigt oder unverzweigt sein und vorzugsweise 1 bis 12, vor allem 1 bis 8 und besonders bevorzugt 1 bis 5 C-Atome enthalten. Beispiele für Alkyl sind Methyl, Aethyl, n-Propyl, Isopropyl, n-Butyl, sek.-Butyl, tert.-Butyl, n-Pentyl, tert.-Amyl, n-Hexyl, n-Heptyl, n-Octyl und 1,1,3,3-Tetramethylbutyl.

Aryl als Bedeutung von A, $R_2$ und Z bedeutet insbesondere unsubstituiertes oder durch Halogen, wie Chlor, $C_1$-$C_6$-Alkyl, wie Methyl, Aethyl,

Isopropyl oder tert.-Butyl, oder $C_1$-$C_6$-Alkoxy, wie Methoxy oder Aethoxy substituiertes Phenyl. Aryl bedeutet bevorzugt unsubstituiertes Phenyl.

X in der Verbindung der Formel III kann als Halogen beispielsweise Chlor, Brom oder Jod, bevorzugt Brom bedeuten.

Im erfindungsgemässen Verfahren verwendet man als Ausgangsstoffe bevorzugt Nitrile der Formel II, worin $R_1$ unsubstituiertes oder substituiertes Phenyl oder Naphthyl bedeutet.

Vor allem verwendet man als Ausgangsstoffe Nitrile der Formel

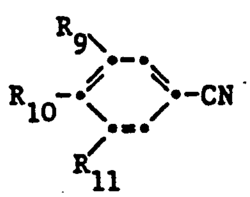

(IV)

worin $R_9$, $R_{10}$ und $R_{11}$ unabhängig voneinander Wasserstoff, Halogen, Cyano, Trifluormethyl, $C_3$-$C_{25}$-Dialkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_3$-$C_{25}$-Dialkanoylamino, $C_2$-$C_{24}$-Dialkylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, N-($C_1$-$C_{12}$)-Alkyl-N-Phenylcarbamoyl oder N-($C_1$-$C_{12}$)-Alkyl-N-benzoylamino bedeuten, wobei mindestens einer der Substituenten $R_9$, $R_{10}$ und $R_{11}$ Wasserstoff bedeutet.

Insbesondere verwendet man als Ausgangsstoffe Nitrile der Formel V

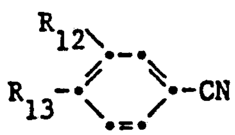

(V)

worin einer der Substituenten $R_{12}$ und $R_{13}$ Chlor, Brom, $C_1$-$C_4$-Alkyl, Cyano, $C_1$-$C_4$-Alkoxy, unsubstituiertes oder durch Chlor oder Methyl substituiertes Phenoxy, $C_3$-$C_9$-Dialkylcarbamoyl, unsubstituiertes oder am Phenyl durch Chlor, Methyl oder Methoxy substituiertes N-($C_1$-$C_4$) -Alkyl-N-phenylcarbamoyl bedeutet, und der andere Wasserstoff ist.

Bevorzugt verwendet man als Ausgangsstoffe Verbindungen der Formel III, worin X Brom oder Chlor, A $C_1$-$C_5$-Alkyl oder Phenyl, und $R_2$ Wasserstoff, $C_1$-$C_5$-Alkyl, Phenyl, Cyano oder die Gruppe -COOZ bedeuten, worin Z $C_1$-$C_5$-Alkyl oder Phenyl ist.

Besonders bevorzugt verwendet man Verbindungen der Formel III, worin X Brom oder Chlor, A Methyl, Aethyl, Isopropyl oder tert.-Butyl, und $R_2$ Wasserstoff, Methyl, Aethyl oder Phenyl bedeuten.

Das erfindungsgemässe Verfahren wird in Gegenwart von Zink durchgeführt. Zweckmässig verwendet man ein aktiviertes Zink. Die Aktivierung des Zinks kann auf in der metallorganischen Chemie übliche Weise erfolgen, beispielsweise durch Behandeln mit einem Kupfersalz, wie Kupferacetat, in Essigsäure, oder mit einer Mineralsäure, wie Salzsäure oder Schwefelsäure.

Man führt die Umsetzung des Esters der Formel III mit dem Nitril in einem inerten organischen Lösungsmittel durch. Inerte Lösungsmittel sind solche, die unter den Reaktionsbedingungen chemisch nicht verändert werden. Als Lösungsmittel eignen sich beispielsweise Aether, wie Tetrahydrofuran oder Dioxan, oder Glykoläther, wie Aethylenglykol-dimethyläther, Aethylenglykol-diäthyläther, Diäthylenglykol-dimethyläther oder Diäthylenglykol-diäthyläther, ferner aliphatische oder aromatische Kohlenwasserstoffe, wie Benzol oder durch Alkyl, Alkoxy oder Halogen substituiertes Benzol, wie Toluol, Xylol, Anisol oder Chlorbenzol. Zudem ist es auch möglich, das umzusetzende Nitril der Formel II gleichzeitig als Lösungsmittel zu verwenden, falls es im Temperaturbereich flüssig ist, in dem die Umsetzung erfolgt. Die genannten Lösungsmittel können auch als Mischungen eingesetzt werden. Zweckmässigerweise verwendet man 1-20 Gew.-Teile Lösungsmittel auf 1 Gew.-Teil der Reaktionsteilnehmer.

Im erfindungsgemässen Verfahren verwendet man bevorzugt einen Aether oder einen aromatischen Kohlenwasserstoff als Lösungsmittel, insbesondere Aethylenglykoldimethyläther, Tetrahydrofuran, Dioxan, Anisol, Benzol, Toluol, o-, m- oder p-Xylol.

Die Umsetzung der Verbindung der Formel II mit der Verbindung der Formel III führt man bei einer Temperatur von weniger als 80°C durch, insbesondere bei 0 bis 80°C, vor allem bei 20 bis 50°C.

Das Reaktionsprodukt wird alkalisch hydrolysiert, indem man die Reaktionslösung beispielsweise mit wässerigem Alkalihydroxid, wie Natrium-, Kalium- oder Lithiumhydroxid, oder wässerigem Erdalkalihydroxid, wie Calcium- oder Magnesiumhydroxid, hydrolysiert. Zur Hydrolyse setzt man die wässerigen Alkali- oder Erdalkalihydroxide bevorzugt in solch einer Menge ein, dass die Reaktionslösung einen pH-Wert von insbesondere 10 bis 14, vor allem 13 bis 14 aufweist.

Nach der Hydrolyse lasst sich der ß-Amino-α,ß-ungesättigte Carbonsäureester der Formel I extrahieren. Zur Extraktion eignen sich mit Wasser nicht mischbare organische Lösungsmittel, wie beispielsweise Diäthyläther, Benzol, Toluol, Methylenchlorid, Chloroform oder Tetrachlorkohlenstoff. Auch andere, dem Fachmann geläufige Aufarbeitungsverfahren sind möglich.

Wenn es auch in der Regel genügt, die Reaktanden in stöchiometrischen Mengen einzusetzen, so kann es sich günstig auf die Ausbeute auswirken,wenn man vor allem das Zink in mehr als stöchiometrischen Mengen, insbesondere bis etwa 2,5 Mol auf 1 Mol der Verbindung der Formel III, verwendet.

Eine bevorzugte Ausführungsform des erfindungsgemässen Verfahrens besteht darin, das Nitril der Formel II zusammen mit dem Metall vorzulegen, und die Verbindung der Formel III zuzudosieren. Die Dosiergeschwindigkeit wird so eingestellt, dass die Temperatur von maximal 80°C nicht überschritten wird.

Die als Ausgangsstoffe verwendeten Verbindungen der Formeln II und III sind bekannt oder lassen sich nach bekannten Verfahren herstellen.

Nach dem erfindungsgemässen Verfahren fallen die Verbindungen der Formel I in der Regel in einer Reinheit an, die deren direkte Weiterverarbeitung erlaubt. Die erfindungsgemäss hergestellten ß-Amino-α,ßungesättigten Carbonsäureester der Formel I stellen nämlich wertvolle Zwischenprodukte dar.

Diese Zwischenprodukte können auf an sich bekannte Weise zur Herstellung von Heterocyclen, wie

Pyridinen (T. Kato et al., Yakugaku Zasshi, 91, 740 (1971); G. Hörlein et al, Liebigs Ann. Chem., 371 (1979)) Pyrimidinen (K. Grohe et al., Liebigs Ann. Chem., 1025 (1973)), Indolen (D. Raileannu et al., Tetrahedron, 27, 5031 (1971)) oder Isothiazolen (R.K. Howe et al., J. Heterocyclic Chem. 15, 1001 (1978)) oder als Thermostabilisatoren für PVC verwendet werden.

Die Verbindungen der Formel I, worin $R_2$ Wasserstoff bedeutet, eignen sich zur Herstellung von Pigmenten, insbesondere von 1,4-Diketopyrrolo-[3,4-c]-pyrrolen, indem man beispielsweise eine Verbindung der Formel I worin $R_2$ Wasserstoff ist, zuerst mit einem Metall oder Metallsalz, dann mit einem aromatischen Nitril und einem Halogenessigsäureester umsetzt.

Die folgenden Beispiele erläutern die Erfindung.

**Beispiel 1**

0,75 g $Cu(OCOCH_3)_2.H_2O$ werden in 25 ml Eisessig bei 90°C gelöst und anschliessend mit 13,1 g Zinkstaub versetzt. Das Gemisch wird 1 Minute gerührt, dekantiert und mit Eisessig und Benzonitril gewaschen. Das Zink wird zu 25 ml Benzonitril gegeben und mit 0,05 g Jod versetzt. Unter Stickstoffatmosphäre tropft man innert 40 Minuten bei 40° C 9,3 ml Bromessigsäuremethylester zu. Nach beendetem Zutropfen wird das Gemisch 5 Stunden bei 40°C gerührt. Dann filtriert man das noch vorhandene Zink ab. Das Filtrat wird mit Eiswasser versetzt und mit wässeriger Natronlauge auf pH 14 gestellt. Man extrahiert das Gemisch mit Chloroform. Die organische Phase wird neutral gewaschen und über Natriumsulfat getrocknet. Nach Einengen im Rotationsverdampfer wird im Hochvakuum destilliert. Man erhält 13,9 g (78 % d.Th. bezogen auf BBromessigsäuremethylester) ß-Aminozimtsäuremethylester, der bei 1,33 Pa bei 105°C siedet.

**Beispiel 2**

105 g Zinkstaub (97,2%-ig) werden gemäss Beispiel 1 in Eisessig mit Kupferacetat aktiviert und in 375 ml Benzonitril vorgelegt. Nach Zugabe von 0,3 g Jod wird unter Rühren eine Mischung aus 83,3 ml Bromessigsäureäthylester und 150 ml Benzonitril innert 1,5 Stunden zugetropft, wobei die Reaktionstemperatur bei 20-25°C gehalten wird. Nach beendetem Zutropfen wird das Gemisch 17 Stunden bei Raumtemperatur weitergerührt. Anschliessend wird von noch vorhandenem Zink abfiltriert. Das Filtrat wird auf 1000 ml Wasser gegossen und mit 30 gew.%-iger wässeriger Natronlauge auf pH 10-11 gestellt und 1 Stunde nachgerührt. Hierauf wird das Gemisch mit 500 ml Diäthyläther versetzt und weitere 30 Minuten gerührt. Die organische Phase wird abgetrennt, mit Wasser neutral gewaschen und über Natriumsulfat getrocknet. Nach Einengen im Rotationsverdampfer wird am Hochvakuum destilliert. Man erhält 91,5 g (64 % der Theorie) ß-Aminozimtsäureäthylester, der bei 1,33 Pa bei 120-125°C siedet.

**Beispiel 3**

Verfährt man wie im Beispiel 2, verwendet jedoch anstelle des aktivierten Zinkes direkt unaktivierten Zinkstaub, so erhält man unter sonst identischer Arbeitsweise in 38-proz. Ausbeute ebenfalls den unter Beispiel 2 beschriebene, β-Aminozimtsäureäthylester.

**Beispiel 4**

210 g Zinkstaub (97,2%-ig) werden in einem Erlenmeyerkolben in 250 ml 2 gew.-%iger wässeriger Salzsäure angeschlämmt und während ca. 2 Minuten immer wieder umgeschüttelt. Anschliessend wird filtriert, der Rückstand mit Wasser neutral gewaschen und hintereinander mit Aethanol, Aceton, Petroläther nachgewaschen und schliesslich bei 100°C im Vakuumschrank getrocknet. Verwendet man im Beispiel 2 dieses mit Salzsäure aktivierte Zink anstelle des mit Eisessig aktivierten Metalls, so erhält man bei analoger Arbeitsweise in 64-proz. Ausbeute β-Aminozimtsäureäthylester.

**Beispiel 5**

Verwendet man im Beispiel 2 anstelle des in Eisessig aktivierten Zinkes feine Zinkspäne, die aus Zinkstäben auf der Drehbank unter Stickstoff frisch abgeraspelt werden, so erhält man bei analoger Reaktionsdurchführung und Aufarbeitung in 63-proz. Ausbeute β-Aminozimtsäureäthylester.

**Beispiel 6**

48,8 g Zinkstaub (97,2%-ig) werden gemäss Beispiel 4 mit Salzsäure aktiviert und in 170 ml Benzonitril vorgelegt. Nach Zugabe von 0,14 g Jod wird eine Mischung aus 51 ml Bromessigsäuretertiärbutylester und 70 ml Benzonitril unter Rühren innert 1 Stunde zugetropft, wobei die Reaktionstemperatur bei 20-25°C gehalten wird. Anschliessend wird 17 Stunden bei Raumtemperatur weitergerührt. Die Aufarbeitung des Reaktionsgemisches gestaltet sich analog Beispiel 2. Man erhält 53,8 g (71% der Theorie) des bei 133 Pa bei 154-155°C siedenden β-Aminozimtsäuretertiärbutylesters. Smp.: 92°C.

**Beispiel 7**

21 g Zinkstaub (97,2%ig) werden gemäss Beispiel 4 mit 2 gew.%-iger wässeriger Salzsäure behandelt. Das so aktivierte Zink wird in 75 ml Diäthylenglykoldimethyläther vorgelegt und mit 0,6 g Jod versetzt. Nun gibt man unter Rühren bei 25°C (eventuell Aussenkühlung) eine Mischung aus 16,7 ml Bromessigsäureäthylester und 15,4 ml Benzonitril zu. Das Gemisch wird 17 Stunden bei Raumtemperatur nachgerührt und anschliessend analog Beispiel 2 basisch aufgearbeitet. Man erhält in 95-%iger Ausbeute den β-Aminozimtsäureäthylester. Ersetzt man im obigen Beispiel das Lösungsmittel Diäthylenglykoldimethyläther durch Toluol bzw. Diäthyläther, so erhält man bei analoger Arbeitsweise ebenfalls gute Ausbeuten an β-Aminozimtsäureäthylester.

**Beispiele 8-12**

Analog Beispiel 7 erhält man weitere β-Aminozimtsäurederivate der allgemeinen Formel

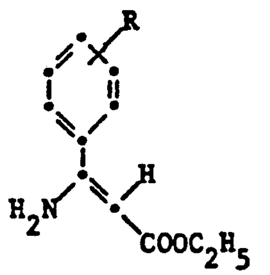

wenn man Bromessigsäureäthylester mit dem entsprechenden in Kolonne 2 der folgenden Tabelle 1 angegebenen Nitril umsetzt. Kolonne 3 gibt die jeweilig erhaltene Ausbeute an.

**Tabelle I**

| Beispiel Nr. | 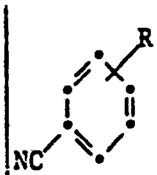 | Ausbeute (% d. Th.) | Smp. | Sdp. |
|---|---|---|---|---|
| 8 | R = p-$CH_3$ | 64 | - | 122 - 123°C bei 10,6 Pa |
| 9 | R = p-Cl | 70 | - | 130 - 132°C bei 10,6 Pa |
| 10 | R = m-Cl | 65 | - | 124 - 125°C bei 8 Pa |
| 11 | R = p-CN | 87 | 83 - 85°C | - |
| 12 | R = m-CN | 76 | 71 - 73°C | - |

**Beispiel 13**

7,2 g Zinkstaub (97,2%-ig) werden gemäss Beispiel 1 in Eisessig mit Kupferacetat aktiviert und in 25 ml Benzonitril vorgelegt. Nach Zugabe von 0,05 g Jod unter Stickstoffatmosphäre tropft man innert 1 Stunde bei 50°C 8,75 ml Chloressigsäuremethylester zu. Nach beendetem Zutropfen wird das Gemisch 15 Stunden bei 65°C gerührt. Dann filtriert man das noch vorhandene Zink ab. Das Filtrat wird mit Eiswasser versetzt und mit wässeriger Natronlauge auf einen pH-Wert von 14 gestellt. Man extrahiert das Gemisch mit Aether. Die ätherische Phase wird neutral gewaschen und über Natriumsulfat getrocknet. Nach anschliessender Destillation erhält man 7.0 g (40 % d.Th. bezogen auf Chloressigsäuremethylester) β-Aminozimtsäuremethylester.

**Beispiel 14**

13,1 g Zinkstaub (97,2%ig) werden gemäss Beispiel 1 in Eisessig mit Kupferacetat aktiviert und in 25 ml α-Cyanothiophen gegeben und mit 0,05 g Jod versetzt. Unter Stickstoffatmosphäre tropft man innert 40 Minuten bei 40°C 9,3 g Bromessigsäuremethylester zu. Nach beendetem Zutropfen wird das Gemisch 5 Stunden bei 40°C gerührt. Dann filtriert man das noch vorhandene Zink ab. Das Filtrat wird mit Eiswasser versetzt und mit wässeriger Natronlauge auf einen pH-Wert von 14 gestellt. Man extrahiert das Gemisch mit Chloroform. Die organische Phase wird neutral gewaschen und über Natriumsulfat getrocknet. Nach Einengen im Rotationsverdampfer wird im Hochvakuum destilliert. Man erhält 9,1 g (50 % d. Theorie bezogen auf Bromessigsäuremethylester) der Verbindung der Formel

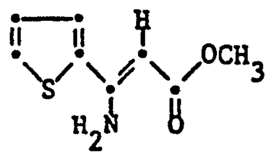

Analyse: Ber. C 52.46 % H 4.92 % N 7.65 %
Gef. C 52.12 % H 4.80 % N 7.41 %

**Beispiel 15**

13,1 g Zinkstaub (97,2%-ig) werden gemäss Beispiel 1 in Eisessig mit Kupferacetat aktiviert und in 30 ml 1,2-Dimethoxyäthan gegeben und mit 0,05 g Jod versetzt. Unter Stickstoffatmosphäre tropft man innert 30 Minuten bei 20°C (Aussenkühlung) 9,3 g Bromessigsäuremethylester zu. Hierauf gibt man innert 30 Minuten 10,4 g 3-Cyano-pyridin hinzu. Das Gemisch wird 6 Stunden bei 40°C gerührt. Anschliessend wird wie im Beispiel 15 aufgearbeitet. Man erhält 4,7 g (43 % d. Theorie bezogen auf Bromessigsäuremethylester) der Verbindung der Formel

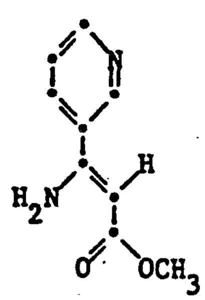

Analyse: Ber. C 60,67 % H 5 61 % N 15 73 %
Gef. C 61,00 % H 5,50 % N 15,50 %

**Beispiele 16-18**

Analog Beispiel 1 erhält man weitere β-Aminozimtsäurederivate der allgemeinen Formel

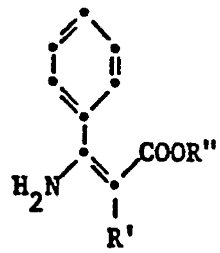

wenn man Benzonitril mit dem entsprechenden in Kolonne 2 der Tabelle 11 angegebenen α-Bromcarbonsäure-Derivat umsetzt. Die Kolonnen 3 und 4 geben die Bedeutung von R′ und R″, und die Kolonne 5 die jeweilig erhaltene Produktausbeute an.

**Tabelle II**

| Beispiel Nr. | Bromcarbonsäure-derivat | R' | R" | Ausbeute (% d.Th.) | Smp. bzw. Sdp. |
|---|---|---|---|---|---|
| 16 | 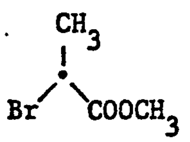 | $CH_3$ | $CH_3$ | 62,5 | 97-98° C bei 0,015 mm |
| 17 | 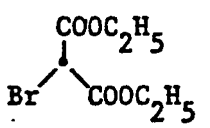 | $C_2H_5$ | $C_2H_5$ | 41 | 108,5° C |
| 18 | 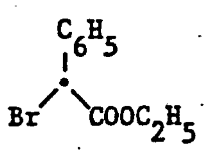 | $C_6H_5$ | $C_2H_5$ | 27 | 69,1° C |

**Beispiel 19**

0,8 g $Cu(OCOCH_3)_2.H_2O$ werden in 25 ml Eisessig bei 90°C gelöst und anschliessend mit 13,1 g Zinkstaub versetzt. Das Gemisch wird 1 Minute gerührt, dekantiert und mit Eisessig und Benzonitril gewaschen. Das Zink wird in 50 ml Benzonitril gegeben und mit 0,05 g Jod versetzt. Man gibt dann 35,4 g β-Amino-p-chlorzimtsäuremethylester zu und rührt während 2 Stunden bei 20 bis 25° C. Diese Mischung versetzt man mit 100 ml p-Xylol und 33,4 g Bromessigsäureäthylester und erwärmt während 10 Stunden bei 130°C. Die Suspension wird abgekühlt, mit 50 ml Aceton versetzt und filtriert. Nach dem Waschen mit Eisessig und Aceton wird getrocknet. Man erhält 12,5 g des Pigmentes der Formel

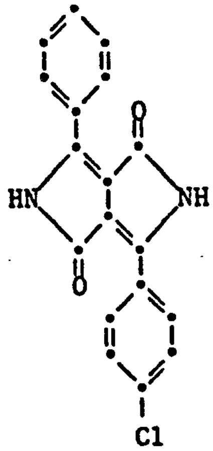

,

das in PVC eingearbeitet eine rote Färbung gibt.
Analyse: $C_{18}H_{11}N_2O_2Cl$ MG 322 5
Ber. C 66,99 % H 3,44 % N 8,68 % C 10,99 %
Gef. C 66,50 % H 3,63 % N 8,54 % C 10,40 %
$\lambda_{max}$ (nm) gemessen in Dimethylformamid: 468/503

**Patentansprüche**

1 Verfahren zur Herstellung von Verbindungen der Formel I

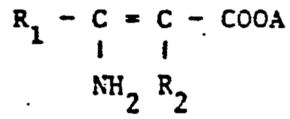

(I),

worin A Alkyl oder Aryl bedeutet, R einen isocyclischen oder heterocyclischen aromatischen Rest bedeutet, $R_2$ Wasserstoff, Alkyl, Aryl, Cyano oder die Gruppe -COOZ bedeutet, worin Z Alkyl oder Aryl ist, indem man 1 Mol eines aromatischen Nitrils der Formel

$$R_1 - CN \text{ (II)}$$

mit 1 Mol eines Essigesters der Formel III

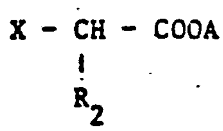

(III),

worin X Halogen bedeutet, in Gegenwart von Zink in einem inerten organischen Lösungsmittel umsetzt, dadurch gekennzeichnet, dass die Umsetzung bei einer Temperatur von weniger als 80°C durchgeführt wird, und dass das entstandene Reaktionsprodukt alkalisch hydrolysiert und die Verbindung der Formel 1 isoliert wird.

2. Verfahren gemäss Anspruch 1, dadurch gekennzeichnet, dass man als Ausgangsstoffe Nitrile der Formel 11 verwendet, worin $R_1$ unsubstituiertes oder substituiertes Phenyl oder Naphthyl bedeutet.

3. Verfahren gemäss Anspruch 1, dadurch gekennzeichnet, dass man Nitrile der Formel IV verwendet,

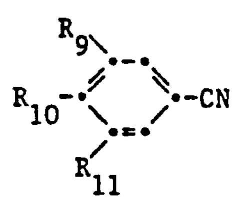

(IV)

worin $R_9$, $R_{10}$ und $R_{11}$ unabhängig voneinander Wasserstoff, Halogen, Cyano, Trifluormethyl, $C_3$-$C_{25}$-Dialkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_3$-$C_{25}$-Dialkanoylamino, $C_2$-$C_{24}$-Dialkylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, N-($C_1$-$C_{12}$)-Alkyl-N-phenylcarbamoyl oder N-($C_1$-$C_{12}$)-Alkyl-N-benzoylaminobedeuten, wobei mindestens einer der Substituenten $R_9$, $R_{10}$ und $R_{11}$ Wasserstoff bedeutet.

4. Verfahren gemäss Anspruch 1, dadurch gekennzeichnet, dass man Nitrile der Formel V verwendet,

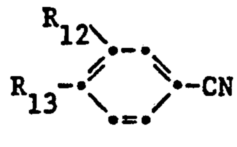

(V)

worin einer der Substituenten $R_{12}$ und $R_{13}$ Chlor, Brom, $C_1$-$C_4$-Alkyl, Cyano, $C_1$-$C_4$-Alkoxy, unsubstituiertes oder durch Chlor oder Methyl substituiertes Phenoxy, $C_2$-$C_5$-Dialkylcarbamoyl, unsubstituiertes oder am Phenyl durch Chlor, Methyl oder Methoxy substituiertes N-($C_1$-$C_4$)-Alkyl-N-phenylcarbamoyl bedeutet, und der andere Wasserstoff ist.

5. Verfahren gemäss Anspruch 1, dadurch gekennzeichnet, dass man als Ausgangsstoffe Verbindungen der Formel 111 verwendet, worin X Brom oder Chlor, A $C_1$-$C_5$-Alkyl oder Phenyl, $R_2$ Wasserstoff, $C_1$-$C_5$-Alkyl, Phenyl, Cyano oder die Gruppe -COOZ bedeuten, worin Z $C_1$-$C_5$-Alkyl oder Phenyl ist.

6. Verfahren gemäss Anspruch 1, dadurch gekennzeichnet, dass man als Ausgangsstoffe Verbindungen der Formel III verwendet, worin X Brom oder Chlor, A Methyl, Aethyl, Isopropyl oder tert.-Butyl, und $R_2$

Wasserstoff, Methyl, Aethyl oder Phenyl bedeuten.

7. Verfahren gemäss Anspruch 1, dadurch gekennzeichnet, dass man aktiviertes Zink verwendet.

8. Verfahren gemäss Anspruch 1, dadurch gekennzeichnet, dass man das Zink in mehr als stöchiometrischen Mengen verwendet.

9. Verfahren gemäss Anspruch 1, dadurch gekennzeichnet, dass man als inertes organisches Lösungsmittel einen aromatischen Kohlenwasserstoff oder einen Aether verwendet.

10. Verfahren gemäss Anspruch 1, dadurch gekennzeichnet, dass man die Umsetzung der Verbindungen der Formel 11 mit der Verbindung der Formel III bei 20 bis 50°C durchführt.

11. Verfahren gemäss Anspruch 1, dadurch gekennzeichnet, dass man die alkalische Hydrolyse bei einem pH von 10 bis 14 durchführt.

12. Verwendung der Verbindungen der Formel 1 gemäss Anspruch 1, soweit $R_2$ Wasserstoff bedeutet, zur Herstellung von 1,4-Diketopyrrolo-[3,4-c]-pyrrolpigmenten.

**Claims**

1. A process for producing a compound of the formula

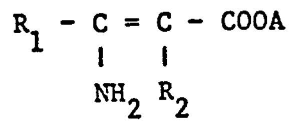

(I)

wherein A is alkyl or aryl, $R_1$ is an isocyclic or heterocyclic aromatic radical, and $R_2$ is hydrogen, alkyl, aryl, cyano or the group -COOZ, in which Z is alkyl or aryl, by reacting 1 mol of an aromatic nitrile of the formula

$R_1$ - CN (II)

with 1 mol of an acetic ester of the formula

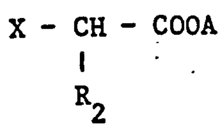

(III)

wherein X is halogen, in the presence of zinc, in an inert organic solvent, which process comprises carrying out the reaction at a temperature of less than 80°C; hydrolysing in an alkaline medium the resultant reaction product; and isolating the compound of the formula I.

2. A process according to claim 1, wherein the starting materials are nitriles of the formula II in which $R_1$ is unsubstituted or substituted phenyl or naphthyl.

3. A process according to claim 1, which comprises the use of nitriles of the formula IV

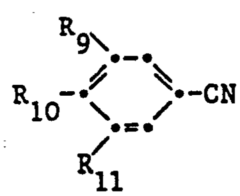

(IV)

wherein $R_9$, $R_{10}$ and $R_{11}$ are each independently hydrogen, halogen, cyano, trifluoromethyl, $C_3$-$C_{25}$-dialkylcarbamoyl, $C_1$-$C_{12}$-alkyl $C_1$-$C_{12}$-alkoxy $C_1$-$C_{12}$-alkylmercapto, $C_2$-$C_{13}$-alkoxycarbonyl $C_3$-$C_{25}$-dialkanoylamino, $C_2$-$C_{24}$-dialkylamino, or are phenoxy, phenylmercapto, phenoxycarbonyl, N-($C_1$-$C_{12}$)-alkyl-N-phenylcarbamoyl or N-($C_1$-$C_{12}$)-alkyl-N-benzoylamino, each unsubstituted or substituted by halogen, $C_1$-$C_{12}$-alkyl or $C_1$-$C_{12}$-alkoxy, at least one of the substituents $R_9$, $R_{10}$ and $R_{11}$ being hydrogen.

4. A process according to claim 1, which comprises the use of nitriles of the formula V

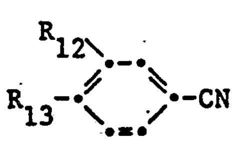

(V)

wherein one of the substituents $R_{12}$ and $R_{13}$ is chlorine, bromine $C_1$-$C_4$-alkyl, cyano, $C_1$-$C_4$-alkoxy, phenoxy which is unsubstituted or substituted by chlorine or methyl, or it is $C_2$-$C_5$-dialkylcarbamoyl, or N-($C_1$-$C_4$)-alkyl-N-phenylcarbamoyl which is unsubstituted or substituted at the phenyl moiety by chlorine, methyl or methoxy; and the other is hydrogen.

5. A process according to claim 1, wherein the starting materials are compounds of the formula III in which X is bromine or chlorine, A is $C_1$-$C_5$-alkyl or phenyl, and $R_2$ is hydrogen, $C_1$-$C_5$-alkyl, phenyl, cyano or the group -COOZ in which Z is $C_1$-$C_5$-alkyl or phenyl.

6. A process according to claim 1, wherein the starting materials are compounds of the formula III in which X is bromine or chlorine, A is methyl, ethyl, isopropyl or tert-butyl, and $R_2$ is hydrogen, methyl, ethyl or phenyl

7. A process according to claim 1, which comprises theuse of activated zinc.

8. A process according to claim 1, wherein the zinc is used in more than stoichiometric amounts.

9. A process according to claim 1, wherein the inert organic solvent is an aromatic hydrocarbon or an ether.

10. A process according to claim 1, wherein the reaction of the compound of the formula II with the compound of the formula III is performed in the temperature range from 20 to 50° C.

11. A process according to claim 1, wherein the alkaline hydrolysis is performed at a pH value of 10 to 14.

12. Use of a compound of the formula I according to claim 1, provided $R_2$ is hydrogen, for producing 1,4-diketopyrrolo-[3,4-c]pyrrole pigments.

**Revendications**

1. Procédé de préparation de commosés répondant à la formule I:

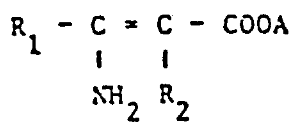

(I)

dans laquelle A représente un alkyle ou un aryle, $R_1$ un radical aromatique isocyclique ou hétérocyclique et $R_2$ l'hydrogène, un alkyle, un aryle, un cyano ou un radical -COOZ dans lequel Z désigne un alkyle ou un aryle, par réaction de 1 mol d'un nitrile aromatique de formule

$R_1$-CN (II)

avec 1 mol d'un ester acétique répondant à la formule III:

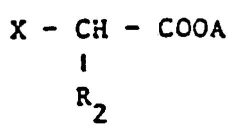

(III)

dans laquelle X represente un halogene, en présence de zinc, dans un solvant organique inerte, procédé caractériaé en ce qu'on effectue la réaction à une température inférieure à 80° C, on hydrolyse en milieu alcalin le produit réactionnel formé et on isole le composé de formule I.

2. Procédé selon la revendication 1, caractérisé en ce qu'on utilise, comme corps de départ, des nitriles de formule II dans lesquels $R_1$ représente un radical phényle ou naphtyle substitué ou non.

3. Procédé selon la revendication 1, caractérisé en ce qu'on utilise des nitriles répondant à la formule IV:

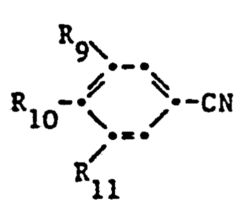

(IV)

dans laquelle $R_9$, $R_{10}$ et $R_{11}$ représentent chacun, indépendamment les uns des autres, l'hydrogène, un halogène, un cyano, un trifluorométhyle, un dialkylcarbamoyle en $C_3$-$C_{25}$, un alkyle en $C_1$-$C_{12}$, un alcoxy en $C_1$-$C_{12}$, un alkylthio en $C_1$-$C_{12}$, un alcoxycarbonyle en $C_2$-$C_{13}$, un dialcanoylamino en $C_3$-$C_{25}$, un dialkylamino en $C_2$-

$C_{24}$, un radical phénoxy, phénylthio ou phénoxycarbonyle non substitué ou porteur d'un halogène, d'un alkyle en $C_1$-$C_{12}$ ou d'un alcoxy en $C_1$-$C_{12}$, un N-alkyl-N-phénylcarbamoyle dont l'alkyle contient de 1 à 12 atomes de carbone ou un N-alkyl-N-benzoylamino dont l'alkyle contient de 1 à 12 atomes de carbone, au moins l'un des symboles $R_9$, $R_{10}$ et $R_{11}$ représentant l'hydrogène.

4. Procédé selon la revendication 1, caractérisé en ce qu'on utilise des nitriles répondant à la formule V:

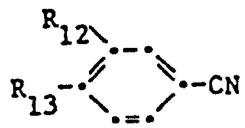

(V)

dans laquelle l'un des symboles $R_{12}$ et $R_{13}$ représente le chlore, le brome, un alkyle en $C_1$-$C_4$, un cyano, un alcoxy en $C_1$-$C_4$, un phénoxy non substitué ou porteur d'un chlore ou d'un méthyle, un dialkylcarbamoyle en $C_2$-$C_5$, ou un N-alkyl-N-phényl-carbamoyle à alkyle en $C_1$-$C_4$, non substitué ou portant, sur son phényle, un chlore, un méthyle ou un méthoxy, et l'autre représente l'hydrogène.

5. Procédé selon la revendication 1, caractérisé en ce qu'on utilise, comme corps de départ, des composés de formule III dans lesquels X représente le brome ou le chlore, A un alkyle en $C_1$-$C_5$ ou un phényle et $R_2$ l'hydrogène, un alkyle en $C_1$-$C_5$, un phényle, un cyano ou un radical -COOZ dont le symbole Z désigne un alkyle en $C_1$-$C_5$ ou un phényle.

6. Procédé selon la revendication 1 caractérisé en ce qu'on utilise, comme corps de départ, des composés de formule III dans lesquels X représente le brome ou le chlore, A un radical méthyle, éthyle, isopropyle ou tertbutyle et $R_2$ l'hydrogène ou un radical méthyle, éthyle ou phényle.

7. Procédé selon la revendication 1, caractérisé en ce qu'on utilise du zinc activé.

8. Procédé selon la revendication 1, caractérisé en ce qu'on utilise le zinc en une quantité supérieure à la quantité stoechiométrique.

9. Procédé selon la revendication 1, caractérisé en ce qu'on utilise. comme solvant organique inerte, un hydrocarbure aromatique ou un éther.

10. Procédé selon la revendication 1, caractérisé en ce qu'on effectue la réaction du composé de formule II avec le composé de formule III à une température comprise 20 et 50° C.

11. Procédé selon la revendication 1, caractérisé en ce qu'on effectue l'hydrolyse alcaline à un pH de 10 à 14.

12. Application de composés de formule I selon la revendication 1 dont le symbole $R_2$ représente l'hydrogène à la préparation de pigments dérivant du dioxo-1,4 pyrrolo[3,4-c]pyrrole.